# EUROPEAN PATENT SPECIFICATION

(11) **EP 4 398 966 B1**
(45) Date of publication and mention of the grant of the patent: **13.05.2026**
(21) Application number: 22786757.9
(22) Date of filing: 09.09.2022
(51) Int. Cl.: A61M 16/04, A61M 16/01

(54) **A PROCEDURAL SEDATION MOUTHPIECE**
PROZEDURALES SEDIERUNGSMUNDSTÜCK
EMBOUT BUCCAL DE SÉDATION PROCÉDURALE

(30) Priority: 10.09.2021 SE 2151118
(43) Date of publication of application: 17.07.2024
(73) Proprietor: Stairway Medical AB, 216 42 Limhamn (SE)
(72) Inventor: ÅKESON, Jonas, 216 42 Limhamn (SE); LJUNGVALL, Magnus, 235 99 Vellinge (SE); BRAIAN, Michael, 235 91 Vellinge (SE); LIST, Thomas, 216 19 Malmö (SE)
(74) Representative: AWA Sweden AB
(86) International application number: PCT/EP2022/075117
(87) International publication number: WO 2023/036935

(56) References cited:
- US-A1- 2010 262 033
- US-A1- 2014 276 171

## Description

### Field of the invention

The present invention relates to procedural sedation and more particularly to a mouthpiece for providing a free upper airway during procedural sedation as defined by the appended claims.

### Background art

Procedural sedation is a technique of administering sedative or dissociative anaesthetic drugs with or without analgesic drugs to induce a state that allows the patient to tolerate unpleasant procedures. It usually involves administration of short-acting sedative drugs.

The demand for procedural sedation has been shown to be increasing, as more and more patients are being offered sedation to reduce discomfort associated with different kinds of medical procedures.

Procedural sedation involves placing the patient in a state of depressed consciousness. In such a state, the patient may be at increased risk of reduced or discontinued respiratory function and cardiac arrhythmias.

Reduced respiratory function during procedural sedation may result from inhibitory effects of the sedative drugs on the respiratory centre in the brain stem, from reduced diaphragmatic activity, and from reduced neuromuscular control of the upper airway tension. Reduced muscle tonus within the oral cavity and throat may cause the lower jaw and tongue to fall backwards and obstruct the upper airways. Such an obstruction must be detected immediately and managed rapidly and appropriately at the bedside to ensure that the patient continues to receive oxygen and remove carbon dioxide. To be prepared for instant such action, anaesthesiologists and anaesthesia nurses constantly monitor the patient for any sign of respiratory depression or hypoxia throughout a procedural sedation. However, procedural sedations are not always performed within the controlled environment of a hospital, but more frequently by less experienced healthcare providers outside hospitals.

It is thus most desirable to find a better way of maintaining patent upper airways during procedural sedation. US2010262033 describes a bite block comprising an insertion channel and an airway channel.

### Summary of the invention

In view of what is stated above, the main objective of the current invention is to provide a mouthpiece that facilitates maintenance of free upper airways during procedural sedation.

To achieve the main objective and also specific objects that will be evident from the following description, a mouthpiece having the features defined in claim 1 is provided according to the present invention. Preferred embodiments of the mouthpiece will be evident from the dependent claims.

More specifically, the present invention provides a mouthpiece for maintenance of a free upper airway during procedural sedation.

The mouthpiece comprises:
a body comprising an airway channel extending from a first end of the body to a second end of the body, where the first end is an anterior end of the body and the second end is a posterior end of the body,
a first stop supported by the body, configured to control the insertion depth of the body into the oral cavity of a patient,
a second stop supported by the body,
a thermoplastic element supported by the body. The thermoplastic element having a malleable state in which the thermoplastic element is configured to receive a dental impression from the patient and a set state for allowing fixation of the dental impression. The thermoplastic element is arranged between the first stop and the second stop such that anterior and posterior movement of the thermoplastic element in relation to the body is restricted by means of the first stop and the second stop, respectively. The position of the jaws of the patient can thus be fixated in the set state of the thermoplastic element, which reduces the risk of the lower jaw and tongue of the patient falling backwards and causing an airway obstruction. This reduces the workload for the caregivers monitoring the patient. The central working and airway channel allows transoral access to the patient for a procedure and enables the patient to breathe unrestricted.

The second stop may comprise a first protrusion extending from the body and having an anterior sloping surface configured to induce anterior movement of the lower jaw of the patient in response to the thermoplastic element receiving the dental impression. The first protrusion facilitates positioning of the lower jaw of the patient in relation to the mouthpiece when the patient forms the dental impression.

The second stop may comprise a second protrusion extending from the body and having an anterior sloping surface configured for appropriately positioning the teeth of the upper and lower jaws in relation to the body in response to the thermoplastic element receiving the dental impression. The upper jaw being correctly arranged in relation to the body facilitates the relative positioning of the lower jaw, which may be positioned in the thermoplastic element after the teeth of the upper jaw have been positioned.

The second stop may comprise a plurality of flanges extending from the body. Those flanges may be arranged with a curvature so that they essentially conform to the shape of the dental arch of the patient.

The first stop may comprise an annular flange associated with the first end of the body.

The mouthpiece may further comprise a grip element supported by the annular flange and configured to facilitate manual handling of the mouthpiece.

The second end of the body may be curved, reducing the discomfort for the patient using the mouthpiece, as interference with the tongue, especially the medial portion thereof, is reduced.

The annular flange may be curved, improving patient comfort as the first stop can thus conform better to the dental arch of the patient.

The mouthpiece may further comprise at least one tubular connection defining a side channel extending from an access port available from the first end of the body into the airway channel. The side channel facilitates monitoring and management of the patient, as the tubular connection may be used for sampling and measurement of carbon dioxide, provision of additional oxygen and/or removal of saliva and/or mucus from the airway channel. The thermoplastic element may be provided with a truncated conical shape tapering towards the second end of the body, facilitating insertion of the mouthpiece into the mouth of the patient.

The thermoplastic element may have a posterior end having a curvature corresponding to that of the first end of the body.

The thermoplastic element may be made of a material chosen from the group consisting of: Ateva^{®} 4030AC, Ateva^{®} 2820AG or Ateva^{®} 1807EG.

The access port of the at least one tubular connection may comprise a fitting of a Luer lock type.

The airway channel may have an elliptical cross-sectional area.

The first stop and the second stop may be integrally formed with the body.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of said element, device, component, means, step, etc., unless explicitly stated otherwise. The steps of any method disclosed herein do not have to be performed in the exact order disclosed, unless explicitly stated.

### Brief description of the drawings

The above, as well as additional objects, features and advantages of the present invention, will be better understood through the following illustrative and non-limiting detailed description of preferred embodiments of the present invention, with reference to the appended drawings, where the same reference numerals will be used for similar elements, wherein:
Figure 1 discloses a front bottom view of a body for a mouthpiece.
Figure 2 discloses a front bottom view of a thermoplastic element for a mouthpiece.
Figure 3 discloses a front perspective view of a mouthpiece.
Figure 4 discloses a rear perspective view of a mouthpiece.
Figure 5 discloses a side view of a body for a mouthpiece.
Figure 6 discloses a side view of a thermoplastic element for a mouthpiece.
Figure 7 discloses a bottom view of a body for a mouthpiece.
Figure 8 discloses a bottom view of a thermoplastic element for a mouthpiece.
Figure 9 discloses a rear perspective view of a body for a mouthpiece.
Figure 10 discloses a rear perspective view of a thermoplastic element for a mouthpiece.

### Description of embodiments

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

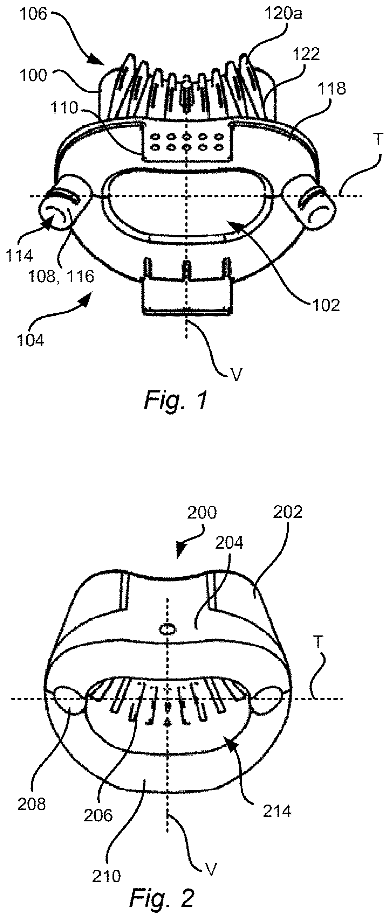

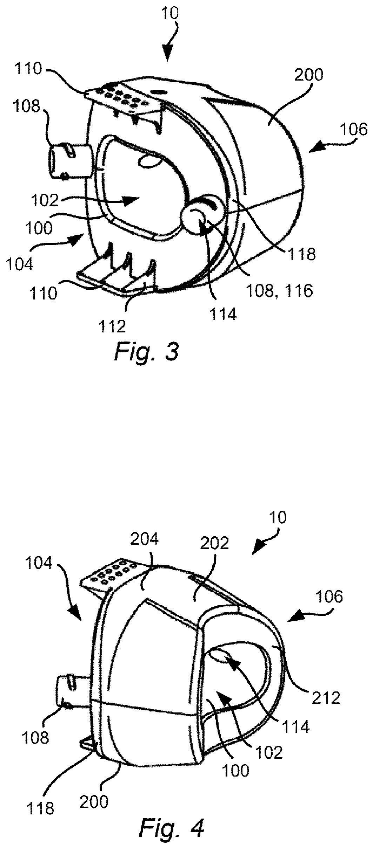

Figure 1 discloses a front bottom view of a body 100 for a mouthpiece 10 (shown in Figures 3 and 4). The body 100 is configured to support a thermoplastic element 200 (shown in Figure 2) and forms together with the thermoplastic element 200 the mouthpiece 10 for providing a free upper airway during procedural sedation.

The body 100 is preferably made in one piece from a plastic material, such as low-density polyethylene (LDPE). The material may be Celanese^{®} AT 220 LDPE or Celanese^{®} AT 7110 LDPE. Other materials having similar properties could also be used. It is however important that the material of the body 100 is approved for medical use. The material of the body 100 may be a thermoplastic material, however, it should preferably have a higher Vicat softening point than that of the thermoplastic element 200.

The material of the body 100 may further be a polypropylene random copolymer, such as Bormed^{™} RF825MO, a polypropylene homopolymer such as Bormed^{™} HG820MO or Bormed^{™} HD850MO etc.

The body 100 comprises an airway channel 102 extending from a first end 104 of the body 100 to a second end 106 of the body 100. The first end 104 is an anterior end of the body 100 and the second end 106 is a posterior end of the body 100.

The second end 106 is intended to be arranged inside the oral cavity of the patient during use of the mouthpiece 10. The airway channel 102 permits not only airway passage but facilitates transoral procedural access, facilitating provision of healthcare for the professional staff taking care of the patient. The airway channel 102 may be provided with an elliptical cross-sectional area. Furthermore, the cross-sectional area of the airway channel 102 may be essentially constant along its length from the first end 104 to the second end 106.

The body further comprises a first stop 118 supported by the body 100 configured to control the insertion depth of the body 100 into the oral cavity of the patient. The first stop 118 is configured to be arranged anteriorly of the teeth of the patient, forming a stop and reducing posterior movement of the body 118 by abutting against the teeth of the patient.

The first stop 118 is preferably integrally formed with the body 100.

The first stop 118 may further in a preferred embodiment comprise an annular flange 118 associated with the first end 104 of the body 100. The first stop 118 further controls/prevents anterior movement of the thermoplastic element 200 in relation to the body 100, and thus prevents the body 100 from moving in a posterior direction in relation to the thermoplastic element 200 when the mouthpiece 10 is arranged in the mouth of the patient.

As is shown in Figure 1, a grip element 110 may be provided supported by the annular flange 118 and configured to facilitate manual handling of the mouthpiece 10. The grip element 110 may comprise an upper and a lower grip element 110, being arranged vertically opposite one another as illustrated in Figure 1. Each grip element 110 preferably extends anteriorly from the annular flange 118 facilitating that a healthcare provider can manipulate and position the mouthpiece 10 as desired in the mouth of the patient before, during and after a procedural sedation.

Each grip element 110 may further be provided with grip enhancing protrusions or ribs, improving the grip on the mouthpiece 10 for the caregiver.

The body 100 supports a second stop 120a, 120b. The second stop 120a, 120b is configured to cooperate with the first stop 118 to secure the thermoplastic element 200 to the body 100 and further to prevent anterior/posterior movement of the thermoplastic element 200 in relation to the body 100. Additionally, the second stop 120a, 120b provides the effect of inducing the desired positoning of the lower jaw of the patient in relation to the upper jaw. The body 100 will be arranged such that the patient bites down into the thermoplastic element 200 with the teeth arranged such that a dental impression is formed in the thermoplastic element 200 between the first stop 118 and the second stop 120a, 120b. The second stop 120a, 120b is thus arranged posteriorly to the teeth of the patient while the first stop 118 is arranged anteriorly to the teeth of the patient.

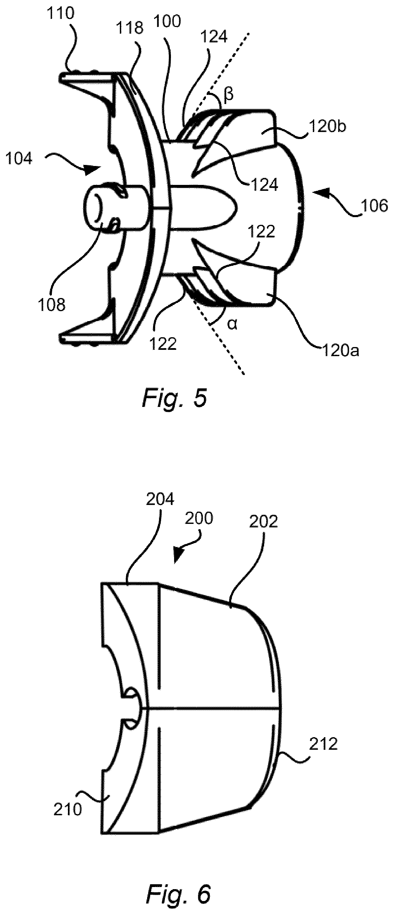

The second stop 120a, 120b may be integrally formed with the body 100. The second stop 120a, 120b may comprise a plurality of flanges extending from the body 100, as is shown in Figure 1 and will be elaborated further on in relation to Figure 5.

Figures 1 and 2 define a transverse direction T and a vertical direction V. The mouthpiece 10 is configured to be arranged in the mouth of the patient such that the transverse direction T of the body 100 and the thermoplastic element 200 extends laterally in relation to the patient while the vertical direction V extends vertically in relation to the patient.

As further illustrated in Figure 1, the body 100 may be provided with at least one tubular connection 108 defining a side channel 114 extending from an access port 116 available from the first end 104 of the body 100 into the airway channel 102. The access port 116 of the at least one tubular connection 108 is preferably a fitting of a Luer lock type. The tubular connection 108 may be used to provide oxygen and/or sample/measure carbon dioxide expired from the patient.

As is shown, the body 100 of the mouthpiece 10 is preferably provided with bilateral tubular connections 108. As a patient during procedural sedation is often positioned with the head in a side position, having bilateral tubular connections 108 allows the uppermost of the tubular connections 108 to be used for efficiently sampling and measuring expired carbon dioxide. The uppermost tubular connection is further less susceptible to being occluded for instance by saliva, which makes it more suitable for sampling and measurement of expired carbon dioxide. The lowermost tubular connection 108 can be used for provision of oxygen, as oxygen is provided with a controlled positive pressure, making it less susceptible to occlusion of the side channel 114 by saliva.

During procedural sedation, it is crucial to maintain continuous oxygenation to reduce the risk of hypoxia and also to measure expired carbon dioxide levels to detect early signs of respiratory depression and reduced spontaneous ventilation.

As is known in the art, and as also mentioned above, the mouthpiece 10 provided herein enables expired levels of carbon dioxide to be measured, for instance by capnography, to provide continuous information on the respiratory function of the patient. Continuous use of capnography during procedural sedation - stated as compulsory by the European Society of Anaesthesiology and Intensive Care (ESAIC) in current clinical guidelines (Eur J Anaesthesiol 2018; 35: 6-24) - is facilitated by the provision of the at least one tubular conneciton 108. The (single or double) tubular connection 108 facilitates compulsory monitoring of the patient during and early after procedural sedation.

During procedural sedation, the oxygenation is continuously monitored with transdermal pulse oximetry, and less often also intermittently with biochemical blood gas analyses in arterial blood to provide estimates of carbon dioxide tension.

Figure 2 discloses a front bottom view of the thermoplastic element 200 for the mouthpiece 10 (shown in Figure 3). The thermoplastic element 200 is configured to be arranged on the body 100 and supported thereby. The thermoplastic element 200 having a malleable state in which the thermoplastic element 200 is configured to receive a dental impression from the patient and a set state for allowing fixation of the dental impression. The thermoplastic element 200 is configurated to be heated to transform the thermoplastic element 200 into a malleable state. The heating is performed to achieve a temperature that is high enough for the thermoplastic element 200 to become plastic and malleable. Placing the thermoplastic element 200 in the malleable state may comprise heating at least the thermoplastic element 200, optionally the entire mouthpiece 10, to a temperature between 40°C and 80°C, preferably between 50°C and 60°C.

Once the thermoplastic element 200 is plastic, i.e. in the malleable state, the mouthpiece 10 provided with the thermoplastic element 200 is placed in the mouth of the patient. The patient's teeth will sink into the thermoplastic element 200 to form a dental impression, which is set in the set state of the thermoplastic element 200 as the thermoplastic element 200 cools down and looses its plasticity. The lower jaw of the patient can thus be fixated in an anterior position with the teeth embedded in the thermoplastic element 200, thereby reducing the risk of the lower jaw falling backwards to a retracted posterior position and obstructing the upper airways.

The material of the thermoplastic element 200 may a ethylene-vinyl acetate (EVA) copolymer. Suitable examples of materials may be Ateva^{®} 4030AC, Ateva^{®} 2820AG or Ateva^{®} 1807EG. Other materials having similar properties could naturally also be used.

However, it has been shown in a test that a combination of a material of the body 100 choosen from the group of Bormed^{™} RF825MO, Bormed^{™} HG820MO or Bormed^{™} HD850MO, and a material of the thermoplastic element 100 chosen from the group of Ateva^{®} 4030AC, Ateva^{®} 2820AG or Ateva^{®} 1807EG, has desired and beneficial properties. A combination of these materials in the body 100 and in the thermoplastic element 200, respectively, achieves desired adhesion and retention of the thermoplastic element 100 to the body 200. This reduces the risk of movement between the two components of the mouthpiece 10, thereby improving fixation of the dental impression and anterior positioning of the lower jaw of the patient.

Especially, the aforementioned materials provide a desired performance in a peeling test according to DIN EN 1464.

The thermoplastic element 200 may comprise an interior channel 214, the internal shape the interior channel 214 preferably corresponds to the external shape of the body 100 and is configured to accommodate the body 100 such that a posterior side of the first stop 118 of the body 100 is arranged against an anterior surface 210 of the thermoplastic element 200.

The thermoplastic element 200 may be provided with at least one side channel 208, preferably bilateral side channels 208, which is configured to accommodate the at least one side channel 108 of the body 100.

Moreover, the thermoplastic element 200 may be provided with at least one recess 206 being configured to accomodate the second stop 120a, 120b of the body 100. The at least one recess 206 may be provided in both the upper and lower portions of the interior channel 214 of the thermoplastic element 200.

Figures 3 and 4 disclose the mouthpiece 10, shown in a front perspective view and in a rear perspective view, respectively. The mouthpiece 10 comprises the body 100 and the thermoplastic element 200 described in the foregoing and is shown in its assembled state in Figures 3 and 4. The thermoplastic element 200 is arranged on the body 100, between the first stop 118 and the second stop 120a, 120b thereof, such that anterior and posterior movement of the thermoplastic element 200 in relation to the body 100 is restricted by means of the first stop 118 and the second stop 120, 120a, 120b respectively.

Figures 5 and 6 show the body 100 and the thermoplastic element 200 of the mouthpiece 10 in a side view. In the embodiment of the body 100 illustrated in Figure 5, the second stop comprises a first protrusion 120a extending from the body 100 and having an anterior sloping surface 122, configured to induce anterior movement of the lower jaw of the patient in response to the thermoplastic element 200 receiving the dental impression. The first protrusion 120a is configured to be arranged facing downwards and is rigid, which causes the teeth of the lower jaw of the patient to be pushed forwards as they sink into the thermoplastic element 200 to form the dental impression. The mouthpiece 10 is to be arranged in the mouth of the patient such that the lower jaw dental impression is formed between the first protrusion 120a and the second stop 118. The inclination angle α of the anterior sloping suface 122 may be between 30° and 90°, preferably between 40° and 60°.

The first protrusion 120a may, as shown in Figure 5, be formed by a plurality of flanges. The first protrusion 120a may comprise between 5 and 12, preferably between 8 and 10, flanges.

In the embodiment shown in Figure 5, the first stop 120a, 120b further comprises a second protrusion 120b extending from the body 100 and having an anterior sloping surface 124 configured for appropriately positioning the teeth of the upper and lower jaws in relation to the body 100 in response to the thermoplastic element 200 receiving the dental impression. The second protrusion 120b is configured to be arranged facing upwards, i.e. toward the upper jaw of the patient, and is rigid, which causes the teeth of the upper jaw of the patient to be pushed forwards in relation to the mouthpiece 10 and the body 100 thereof as they sink into the thermoplastic element 200 to form the dental impression. The mouthpiece 10 is to be arranged in the mouth of the patient such that the upper jaw dental impression is formed between the second protrusion 120b and the second stop 118. The inclination angle β of the anterior sloping suface 124 may be between 30° and 90°, preferably between 40° and 60°.

The second protrusion 120b may as is shown in Figure 5 be formed by a plurality of flanges. The second protrusion 120b may comprise between 5 and 12 flanges, preferably between 8 and 10 flanges.

The mouthpiece 10 may be configured to be arranged in the mouth of the patient such that the upper jaw dental impression is formed before the lower jaw dental impression.

In a preferred embodiment, the body 100 is symmetrical both laterally and vertically, such that the mouthpiece 10 can be arranged with either the first protrusion 120a or the second protrusion 120b facing upwards (i.e. cranially) in relation to the patient.

An embodiment in which the first protrusion 120a is arranged anteriorly of the second protrusion 120b is also envisioned. In such an embodiment, the first protrusion 120a would induce increased anterior movement of the lower jaw of the patient in comparison to the embodiment shown in Figure 5, in which the first and second protrusions 120a, 120b are arranged directly above/below each other.

Figure 6 shows that the thermoplastic element 200 may be provided with a truncated conical shape tapering towards the second end 106 of the body 100, i.e. towards a posterior end 212 of the thermoplastic element 200. The thermoplastic element 200 may thus be provided with a conical surface 202. The conical shape of the thermoplastic element 200 facilitates insertion of the mouthpiece 200 into the mouth of the patient and reduces discomfort.

The thermoplastic element 200 may furhter be provided with a cylindrical circumferential surface 204, which is arranged between the anterior surface 210 and the circumferential conical surface 202. The cylindrical circumferential surface 204 provides a surface onto which the dental impression may be formed, and which is directed at a close-to perpendicular angle to the teeth of the patient. This facilitates forming the dental impression and reduces the risk that the mouthpiece 10 slides in an anterior direction in relation to the patient as the dental impression is formed in the thermoplastic element 200.

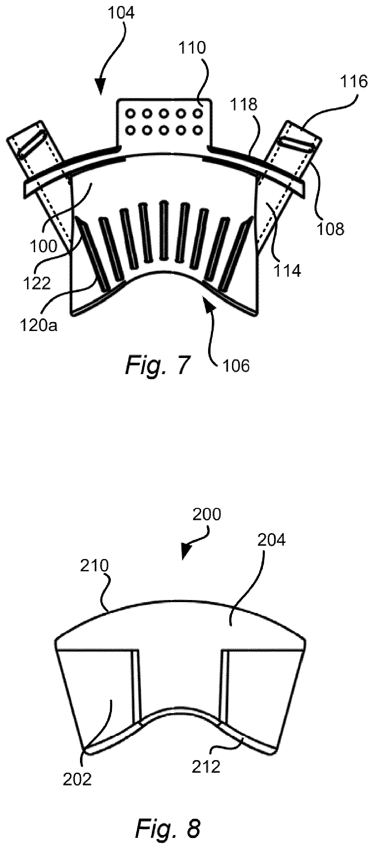

Figures 7 and 8 show the body 100 and the thermoplastic element 200 of the mouthpiece 10 in a bottom view. As shown in Figure 7, the second end 106 of the body 100 may be curved. The curvature of the posterior end 106 of the mouthpiece 10 extends laterally and reduces interference of the mouthpiece 10 with the tongue of the patient.

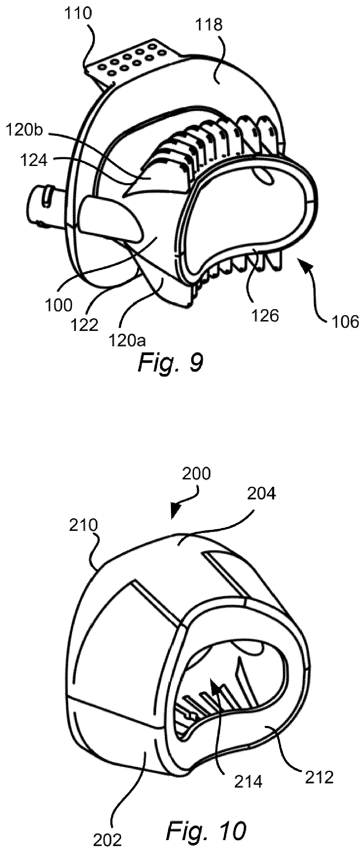

The posterior end 212 of the thermoplastic element 200 may be configurated to cover a posterior surface 126 of the body 100 (shown in Figure 9). The posterior end 212 of the thermoplastic element 200 may thus form a posterior surface of the mouthpiece 10. The posterior end 212 of the thermoplastic element 200 may further be curved and conforming to the curvature of the second end 106 of the body 100. The curvature of the second end 106 of the body 100 and of the posterior end 212 of the thermoplastic element 200 improves patient comfort during use of the mouthpiece 10, while the mouthpiece 10 still provides sufficient contact with the tongue to reduce the risk of it obstructing the airway channel 102. The mouthpiece 10 will by the curvature described in the foregoing have a posterior extension into the mouth that is less medially on the tongue and is as longest on the lateral sides of the tongue of the patient, which reduces discomfort for the patient.

Figures 7 and 8 further exemplify that the annular flange 118 of the body 100 may be curved and correspondingly that the anterior surface 210 of the thermoplastic element 200 may be provided with a corresponding curvature. The curvature of the annular flange 118 allows the mouthpiece 10 to conform to the shape of the dental arch of the patient and improves the comfort for the patient using the mouthpiece 10.

Additionally, Figure 7 shows how the first protrusion 120a may be provided with a corresponding curvature to that of the annular flange 118. The lateral curvature of the first protrusion 120a is intended to conform to the dental arch of the patient, such that pressure from forming the dental impression of the patient in the mouthpiece 10 is distributed more evenly over the first protrusion 120a. The flanges of the first protrusion 120a may be arranged such that the anterior sloping surface 122 on each of the flanges is arranged to form the aforementioned curvature. Furthermore, the second protrusion 120b may also be provided with a curvature as described in the foregoing in conjunction with the first protrusion 120a.

Furthermore, each side channel 114 may be arranged such that it extends at an angle in relation to the extension of the airway channel 102. Each side channel 114 extends from the access port 116 in both a lateral and posterior direction into the airway channel 102. When the patient is placed in a side position, the upper side channel 114 enables carbon dioxide to be sampled and measured, and the lower side channel 114 enables oxygen to be supplied and/or saliva to be drained.

Figures 9 and 10 show the body 100 and the thermoplastic element 200 in a rear perspective view. Figure 9 shows how the body 100 at the second end 106 thereof may be provided with a curved posterior surface 126, the curvature of which is intended to conform to the curvature of the posterior end 212 of the thermoplastic element 200.

It will be appreciated that the present invention is not limited to the embodiments shown. Several modifications and variations are thus conceivable within the scope of the invention, which is thus exclusively defined by the appended claims.

## Claims

1. A mouthpiece (10) for providing a free upper airway during procedural sedation, the mouthpiece (10) comprising:
a body (100) comprising an airway channel (102) extending from a first end (104) of the body (100) to a second end (106) of the body (100), wherein the first end (104) is an anterior end of the body (100) and the second end (106) is a posterior end of the body (100),
a first stop (118) supported by the body (100) configured to control the insertion depth of the body (100) into the oral cavity of a patient,
a second stop (120a, 120b) supported by the body (100),
a thermoplastic element (200) supported by the body (100), the thermoplastic element (200) having a malleable state in which the thermoplastic element (200) is configured to receive a dental impression from the patient and a set state for allowing fixation of the dental impression, wherein the thermoplastic element (200) is arranged between the first stop (118) and the second stop (120a, 120b) such that anterior and posterior movement of the thermoplastic element (200) in relation to the body (100) is restricted by means of the first stop (118) and the second stop (120, 120a, 120b), respectively,
wherein the second stop (120a, 120b) comprises a first protrusion (120a) extending from the body (100) and having an anterior sloping surface (122) configured to induce anterior movement of the lower jaw of the patient in response to the thermoplastic element (200) receiving the dental impression.

2. The mouthpiece (10) according to claim 1, wherein the second stop (120a, 120b) comprises a second protrusion (120b) extending from the body (100) and having an anterior sloping surface (124) configurated for appropriately positioning the teeth of the upper and lower jaws in relation to the body (100) in response to the thermoplastic element (200) receiving the dental impression.

3. The mouthpiece (10) according to any one of claims 1 or 2, wherein the second stop (120a, 120b) comprises a plurality of flanges extending from the body (100).

4. The mouthpiece (10) according to any of the preceding claims, wherein the first stop (118) comprises an annular flange (118) associated with the first end (104) of the body (100).

5. The mouthpiece (10) according to claim 4, further comprising a grip element (110) supported by the annular flange (118) and configurated to facilitate manual handling of the mouthpiece (10).

6. The mouthpiece (10) according to any one of the preceding claims, wherein the second end (106) of the body (100) is curved.

7. The mouthpiece according to any one of the preceding claims, wherein the annular flange (118) is curved.

8. The mouthpiece (10) according to any one of the preceding claims, further comprising at least one tubular connection (108) defining a side channel (114) extending from an access port (116) available from the first end (104) of the body (100) into the airway channel (102).

9. The mouthpiece (10) according to any one of the preceding claims, wherein the thermoplastic element (200) has a truncated conical shape tapering towards the second end (106) of the body (100).

10. The mouthpiece (10) according to claim 9 when dependent on claim 6, wherein the thermoplastic element (200) has a posterior end (212) having a curvature corresponding to that of the first end (104) of the body (100).

11. The mouthpiece (10) according to any one of the preceding claims, wherein the thermoplastic element (200) is made of ethylene-vinyl acetate (EVA) copolymer.

12. The mouthpiece (10) according to claim 8, wherein the access port (116) of the at least one tubular connection (108) comprises a fitting of a Luer lock type.

13. The mouthpiece (10) according to any one of the preceding claims, wherein the airway channel (102) has an elliptical cross-sectional area.

14. The mouthpiece (10) according to any one of the preceding claims, wherein the first stop (118) and the second stop (120a, 120b) are integrally formed with the body (100).

## Patentansprüche

1. Mundstück (10) zum Bereitstellen eines freien oberen Atemwegs während prozeduraler Sedierung, wobei das Mundstück (10) umfasst:
einen Körper (100) umfassend einen Atemwegkanal (102), der von einem ersten Ende (104) des Körpers (100) zu einem zweiten Ende (106) des Körpers (100) verläuft, wobei das erste Ende (104) ein anteriores Ende des Körpers (100) ist und das zweite Ende (106) ein posteriores Ende des Körpers (100) ist,
einen ersten Anschlag (118), der von dem Körper (100) getragen wird und dafür gestaltet ist, die Einführtiefe des Körpers (100) in die Mundhöhle eines Patienten zu steuern,
einen zweiten Anschlag (120a, 120b), der von dem Körper (100) getragen wird,
ein thermoplastisches Element (200), das von dem Körper (100) getragen wird, wobei das thermoplastische Element (200) einen formbaren Zustand, in dem das thermoplastische Element (200) dafür gestaltet ist, einen Zahnabdruck des Patienten aufzunehmen, und einen verfestigten Zustand, um Fixieren des Zahnabdrucks zu ermöglichen, aufweist, wobei das thermoplastische Element (200) zwischen dem ersten Anschlag (118) und dem zweiten Anschlag (120a, 120b) angeordnet ist, so dass anteriore und posteriore Bewegung des thermoplastischen Elements (200) bezogen auf den Körper (100) durch den ersten Anschlag (118) bzw. den zweiten Anschlag (120, 120a, 120b) begrenzt wird,
wobei der zweite Anschlag (120a, 120b) einen ersten Vorsprung (120a) umfasst, der sich von dem Körper (100) erstreckt und eine anteriore geneigte Oberfläche (122) aufweist, die dafür gestaltet ist, anteriore Bewegung des Unterkiefers des Patienten als Reaktion darauf zu induzieren, dass das thermoplastische Element (200) den Zahnabdruck aufnimmt.

2. Mundstück (10) nach Anspruch 1, wobei der zweite Anschlag (120a, 120b) einen zweiten Vorsprung (120b) umfasst, der sich von dem Körper (100) erstreckt und eine anteriore geneigte Oberfläche (124) aufweist, die dafür gestaltet ist, die Zähne des Ober- und des Unterkiefers bezogen auf den Körper (100) als Reaktion darauf, dass das thermoplastische Element (200) den Zahnabdruck aufnimmt, geeignet zu positionieren.

3. Mundstück (10) nach einem der Ansprüche 1 oder 2, wobei der zweite Anschlag (120a, 120b) eine Vielzahl von Flanschen umfasst, die sich von dem Körper (100) erstrecken.

4. Mundstück (10) nach einem der vorstehenden Ansprüche, wobei der erste Anschlag (118) einen ringförmigen Flansch (118) umfasst, der mit dem ersten Ende (104) des Körpers (100) verbunden ist.

5. Mundstück (10) nach Anspruch 4, ferner umfassend ein Griffelement (110), das von dem ringförmigen Flansch (118) getragen wird und dafür gestaltet ist, manuelle Handhabung des Mundstücks (10) zu erleichtern.

6. Mundstück (10) nach einem der vorstehenden Ansprüche, wobei das zweite Ende (106) des Körpers (100) gekrümmt ist.

7. Mundstück nach einem der vorstehenden Ansprüche, wobei der ringförmige Flansch (118) gekrümmt ist.

8. Mundstück (10) nach einem der vorstehenden Ansprüche, ferner umfassend wenigstens eine rohrförmige Verbindung (108), die einen Seitenkanal (114) definiert, der sich von einer Zugangsöffnung (116) erstreckt, die vom ersten Ende (104) des Körpers (100) in den Atemwegkanal (102) verfügbar ist.

9. Mundstück (10) nach einem der vorstehenden Ansprüche, wobei das thermoplastische Element (200) eine kegelstumpfförmige Form aufweist, die sich in Richtung zu dem zweiten Ende (106) des Körpers (100) verjüngt.

10. Mundstück (10) nach Anspruch 9, wenn abhängig von Anspruch 6, wobei das thermoplastische Element (200) ein posteriores Ende (212) mit einer Krümmung aufweist, die jener des ersten Endes (104) des Körpers (100) entspricht.

11. Mundstück (10) nach einem der vorstehenden Ansprüche, wobei das thermoplastische Element (200) aus Ethylen-Vinylacetat(EVA)-Copolymer besteht.

12. Mundstück (10) nach Anspruch 8, wobei die Zugangsöffnung (116) der wenigstens einen rohrförmigen Verbindung (108) ein Anschlussstück eines Luer-Lock-Typs umfasst.

13. Mundstück (10) nach einem der vorstehenden Ansprüche, wobei der Atemwegskanal (102) eine elliptische Querschnittsfläche aufweist.

14. Mundstück (10) nach einem der vorstehenden Ansprüche, wobei der erste Anschlag (118) und der zweite Anschlag (120a, 120b) integral mit dem Körper (100) gebildet sind.

## Revendications

1. Embout buccal (10) destiné à maintenir les voies respiratoires supérieures dégagées lors d'une sédation interventionnelle, l'embout buccal (10) comprenant :
un corps (100) comprenant un canal respiratoire (102) s'étendant d'une première extrémité (104) du corps (100) jusqu'à une seconde extrémité (106) du corps (100), dans lequel la première extrémité (104) est une extrémité antérieure du corps (100) et la seconde extrémité (106) est une extrémité postérieure du corps (100),
une première butée (118) supportée par le corps (100) configurée pour contrôler la profondeur d'insertion du corps (100) dans la cavité buccale d'un patient,
une seconde butée (120a, 120b) supportée par le corps (100),
un élément thermoplastique (200) supporté par le corps (100), l'élément thermoplastique (200) présentant un état malléable dans lequel l'élément thermoplastique (200) est configuré pour recevoir une empreinte dentaire du patient et un état durci pour permettre la fixation de l'empreinte dentaire, l'élément thermoplastique (200) étant agencé entre la première butée (118) et la seconde butée (120a, 120b) de telle sorte que les mouvements antérieur et postérieur de l'élément thermoplastique (200) par rapport au corps (100) soient limités au moyen de la première butée (118) et de la seconde butée (120, 120a, 120b), respectivement,
dans lequel la seconde butée (120a, 120b) comprend une première protubérance (120a) s'étendant à partir du corps (100) et ayant une surface antérieure inclinée (122) configurée pour induire un mouvement antérieur de la mâchoire inférieure du patient en réponse à la réception, par l'élément thermoplastique (200), de l'empreinte dentaire.

2. Embout buccal (10) selon la revendication 1, dans lequel la seconde butée (120a, 120b) comprend une seconde protubérance (120b) s'étendant à partir du corps (100) et ayant une surface antérieure inclinée (124) configurée pour positionner de manière appropriée les dents des mâchoires supérieure et inférieure par rapport au corps (100) en réponse à la réception, par l'élément thermoplastique (200), de l'empreinte dentaire.

3. Embout buccal (10) selon l'une quelconque des revendications 1 ou 2, dans lequel la seconde butée (120a, 120b) comprend une pluralité de brides s'étendant à partir du corps (100).

4. Embout buccal (10) selon l'une quelconque des revendications précédentes, dans lequel la première butée (118) comprend une bride annulaire (118) associée à la première extrémité (104) du corps (100).

5. Embout buccal (10) selon la revendication 4, comprenant en outre un élément de préhension (110) supporté par la bride annulaire (118) et configuré pour faciliter la manipulation manuelle de l'embout buccal (10).

6. Embout buccal (10) selon l'une quelconque des revendications précédentes, dans lequel la seconde extrémité (106) du corps (100) est incurvée.

7. Embout buccal selon l'une quelconque des revendications précédentes, dans lequel la bride annulaire (118) est incurvée.

8. Embout buccal (10) selon l'une quelconque des revendications précédentes, comprenant en outre au moins un raccord tubulaire (108) définissant un canal latéral (114) s'étendant à partir d'un orifice d'accès (116) disponible depuis la première extrémité (104) du corps (100) dans le canal de voies respiratoires supérieures (102).

9. Embout buccal (10) selon l'une quelconque des revendications précédentes, dans lequel l'élément thermoplastique (200) a une forme tronconique se rétrécissant vers la seconde extrémité (106) du corps (100).

10. Embout buccal (10) selon la revendication 9 prise en dépendance de la revendication 6, dans lequel l'élément thermoplastique (200) a une extrémité postérieure (212) ayant une courbure correspondant à celle de la première extrémité (104) du corps (100).

11. Embout buccal (10) selon l'une quelconque des revendications précédentes, dans lequel l'élément thermoplastique (200) est en copolymère éthylène/acétate de vinyle (EVA) .

12. Embout buccal (10) selon la revendication 8, dans lequel l'orifice d'accès (116) de l'au moins un raccord tubulaire (108) comprend un raccord du type Luer Lock.

13. Embout buccal (10) selon l'une quelconque des revendications précédentes, dans lequel le canal respiratoire (102) a une section transversale elliptique.

14. Embout buccal (10) selon l'une quelconque des revendications précédentes, dans lequel la première butée (118) et la seconde butée (120a, 120b) sont formées d'un seul tenant avec le corps (100).
